# EUROPEAN PATENT SPECIFICATION

(11) **EP 1 453 461 B1**
(45) Date of publication and mention of the grant of the patent: **01.08.2012**
(21) Application number: 02791909.1
(22) Date of filing: 13.12.2002
(51) Int. Cl.: A61G 5/12, A61F 5/02, A47C 7/46

(54) **TORSO SUPPORT STRUCTURES**
TRÄGERSTRUKTUREN FÜR OBERKÖRPER
STRUCTURES DE SUPPORT DU TORSE

(30) Priority: 14.12.2001 GB 0129931; 14.05.2002 GB 0211002
(43) Date of publication of application: 08.09.2004
(73) Proprietor: The Helping Hand Company (Ledbury) Limited, Ledbury, Herefordshire HR8 1NS (GB)
(72) Inventor: MOORE, Stuart, Ledbury, Hertfordshire HR8 2DZ (GB); GOLDSMITH, John Edward, Tamworth, Staffordshire B78 3AD (GB)
(74) Representative: Fyles, Julie Marie
(86) International application number: PCT/GB2002/005687
(87) International publication number: WO 2003/051264

(56) References cited:
- WO-A-91/16874
- WO-A-96/12465
- WO-A-96/39110
- DE-A- 3 937 911
- DE-C- 19 915 719
- NL-C- 1 016 775
- US-A- 4 367 897
- US-A- 4 874 203
- US-A- 5 228 747

## Description

Patients who suffer from muscular or skeletal defects or who are recovering from serious injuries or operations may need to be provided with substantial physical support, particularly for the upper torso, to enable them to maintain a seated attitude.

US5228747 discloses an upper torso support structure. It is an object of this invention to provide an upper torso support structure which will provide such support and which is adjustable to suit the requirements of particular patients.

According to the invention there is provided an upper torso support structure comprising a central spine and at least two left and right pairs of flexible ribs curving round In front of the central spine, the ribs being capable of being set in desired attitudes to restrict movement of a patient whose torso is held by the support structure.

Each pair of ribs is slidably located onto the spine to enable the pair of ribs to be moved lengthwise across the spine, thus varying the respective lengths of the two ribs as they project to the left and right sides of the spine.

It is of advantage that the pairs of ribs should be mounted so as to be able to slide adjustably towards and away from the spine or be capable of rotation with respect to the spine. It is also preferred that the pairs of ribs should be mounted so as to be able to slide adjustably up and down the spine.

The upper end of the spine ideally carries a head support for the head of the patient. The head support can comprise a head rest with one or two left and right pairs of flexible ribs curving round in front of the head rest, the ribs being settable in desired attitudes to restrict movement of the head of a patient with respect to the head rest. Ideally the head rest will be tiltable and/or rotatable with respect to the spine.

In the preferred form each pair of ribs will be in the form of a strip. Ideally each pair of ribs could be provided with a flexible strip of material lying parallel thereto, with a releasable locking arrangement being provided to lock each rib of the pair to its strip to aid in the fixing of the ribs against movement.

The ribs and/or the flexible strips could be formed from bendable aluminium or thin steel. The material for the ribs is ideally selected so as to allow for some resilience from a set position, but with "memory" such that it will tend to return to the set position after flexing. The same characteristics can also apply to the material from which the spine is formed, which material could comprise a robust flexible composite of plastics material or a glassfibre material. As an alternative each rib could comprise a row or rows of beads strung on a cable, the beads having non-straight through-passageways, together with a tensioning member to act to tension the cable within the passageways in the beads so as to lock the row of beads in a desired attitude.

It is highly preferable that the ribs are provided with external padding. The base of the spine may be attached to a seat platform, preferably such that the spine can be tilted forwards and backwards with respect to the seat. In one arrangement it is also of advantage that the spine is connected to the platform such that it can be rotated about the seat platform. Alternatively or additionally mountings for each pair of ribs could be slidably carried by horizontally curved bars forming parts of the spine.

Ideally the support structure will be provided with mountings enabling it to be attached adjustably to an existing seat. Additionally or alternatively, pivoted legs can extend down from the front edge of the seat platform. In a preferred construction support members, to which said legs are pivoted, are slidably received within slots in the body of the seat platform. Height-adjustable and/or rotatable and/or laterally slidable footrests can be carried by said legs.

The support structure can also incorporate hip support members, adjustable in position, which project forwards from the sides of the lower end of the spine. There may also be shoulder support members mounted near the upper end of the spine. The shoulder supports can incorporate rotatably adjustable end sections.

The invention may be performed in various ways and a preferred embodiment thereof will now be described, by way of example, with reference to the accompanying drawings, in which:-
Figure 1 is a generalised illustration of one form of a torso support structure of this invention;
Figure 2 is an underneath perspective view of the structure shown in Figure 1;
Figure 3 is a rear view of the structure shown in Figures 1 and 2;
Figure 4 shows a detail of a modification of part of the structure of Figure 1;
Figures 5 and 6 are rear and side views respectively of an alternative form of torso support structure of this invention;
Figure 7 illustrates the arrangement in the side view of Figure 6, but in an alternative attitude;
Figure 8 is a plan view of the arrangement of Figures 5 and 6; and
Figures 9, 10 and 11 are rear, front and side views respectively of a further example of a torso support structure of this invention.

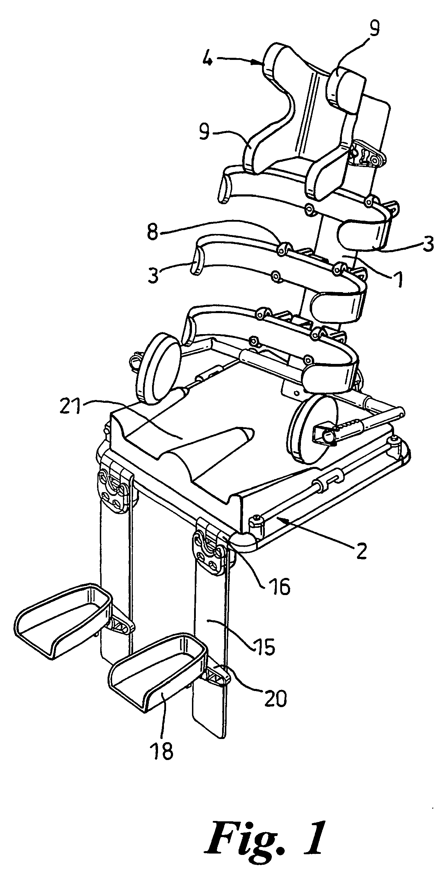

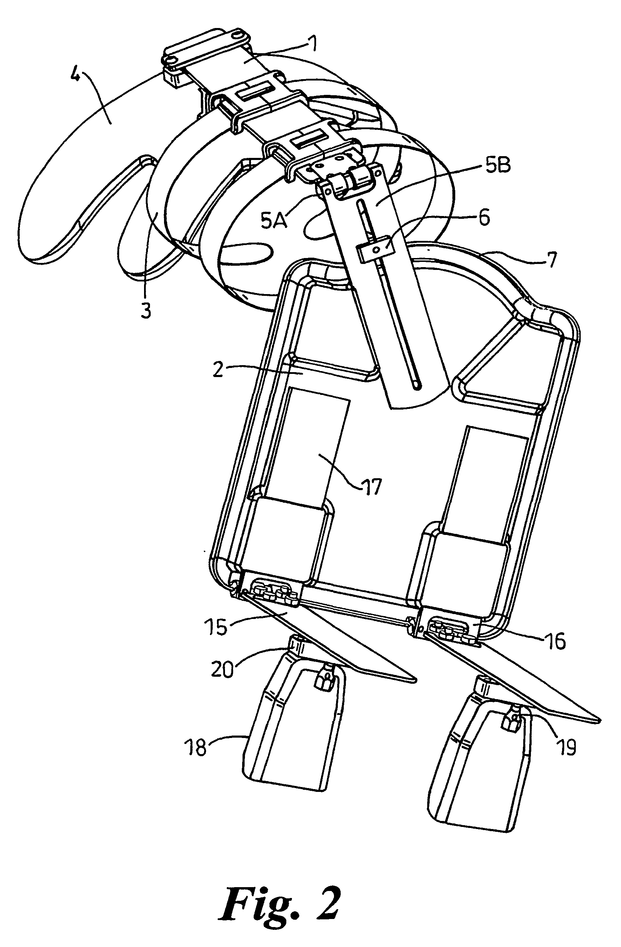

The main part of the support structure shown in Figure 1 comprises a spine 1 pivotally attached to a seat 2 and carrying support ribs 3 and a head support 4. The purpose of this mechanism is that a patient can be positioned on the seat 2 and then the location of the ribs 3 and the headrest 4 can be modified to suit the particular attitude of the patient who is to be supported. The vertical attitude of the patient can be adjusted by pivoting the spine 1 about a friction hinge 5A (shown in more detail in the other drawings). There can be a locking mechanism to hold the spine 1 in a desired attitude. Furthermore, as can be seen from Figure 2, a support plate 5B for the hinged spine 1 is slidable in and out with respect to a mounting plate 6. Additionally the mounting plate 6, carrying the support plate 5B, can be rotated about an arcuate bar 7 with respect to the seat 2.

The ribs 3 themselves are slidably mounted in brackets 8 on the spine 1 such that the rib ends forming a pair of ribs 3 can be modified in length with respect to one another. The ribs 3 are formed from flexible aluminium sheet which can be bent into a desired attitude and will hold the positions into which they are formed. By suitable adjustment of the location and attitude of the ribs 3 and the positioning and tilt of the spine 1 it is possible to ensure that a patient can be held securely in an attitude which is most comfortable for him.

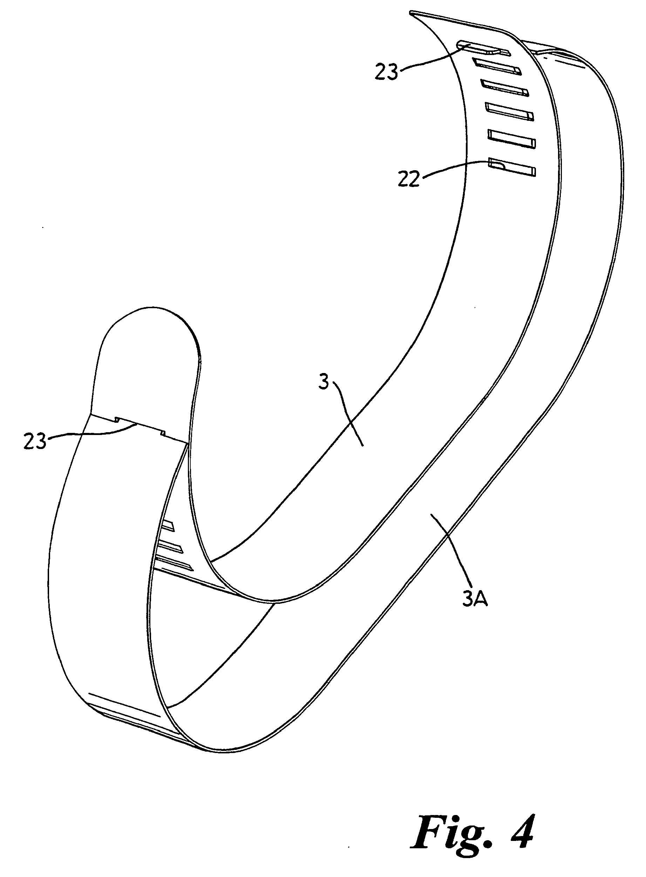

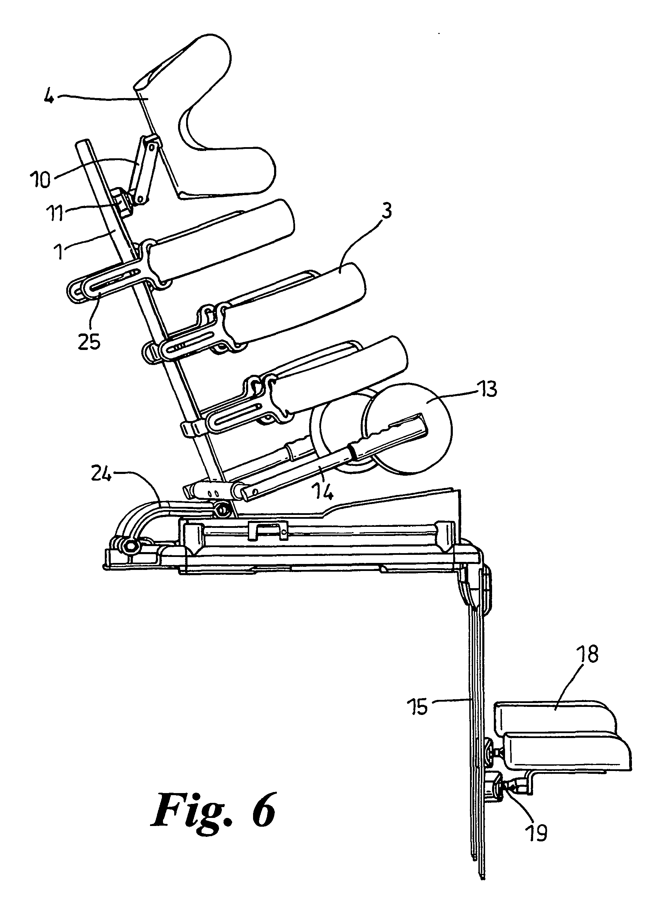

For the ribs 3 in particular it is possible to provide flexible strips 3A (Figure 4) lying parallel to these ribs. The flexible strips can also be distorted into an attitude, comparable to that for the ribs 3 (as they are adjusted to suit the comfort of the patient). Releasable locking arrangements in the form of slots 22 and tabs 23 are provided to lock the ends of the strip 3A to the ends of the rib 3 (as shown in Figure 6). This will provide significant resistance to any tendency for the ribs 3 to be bent back out of the desired position, due to movement of the patient. An alternative locking arrangement could be achieved by using Velcro-type (Registered Trademark) releasable interconnecting parts instead of the tabs 23 and slots 22. The same principle could be applied to ribs 9 of the headrest 4.

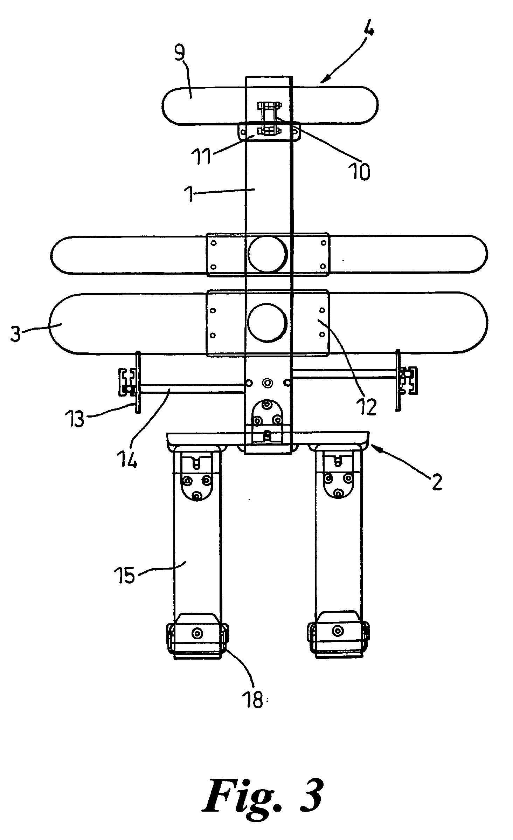

Additionally, support is provided for the head of the patient by means of the headrest 4. Again this is provided with pairs of flexible ribs 9 which can be bent into an attitude suitable to support the shape of the head of the patient. Further adjustment is provided by the manner in which the headrest 4 is mounted on to the spine 1. The arrangement shown in Figure 3 of the drawings is essentially the same as that shown in Figures 1 and 2 although there are certain modifications in detail to illustrate alternative structures or particular features of the whole arrangement. Thus Figure 3 shows a headrest 4 in the form of just one pair of ribs 9. The headrest 4 is mounted on an articulated arm 10, which allows for rotation and alignment of the headrest into a desired position. A mounting 11 of the joint 10 onto the spine 1 can be slid up and down the spine 1 and then locked into a desired position. The arrangement of Figure 3 also shows a modified structure for supporting the ribs 3 in the form of sleeves 12. Another feature shown in Figures 1 and 3 is the provision of adjustable hip support pads 13. These are carried on height-adjustable arms 14 connected to the spine 1, in such a manner that the pads 13 can be rotated and moved forwards and backwards along the arms 14 into positions which will be most comfortable for supporting the hips of a patient.

The headrest incorporating the ribs 9 and the hip support pads 13 will all be provided with suitable external padding to provide for comfort where they come into contact with the body of the patient.

The seat 2 is also provided with legs 15 pivoted about hinges 16. The hinges 16 are carried by support plates 17 (Figure 2) which slide within the body of the seat 2 so that the forward location of each of the legs 15 with respect to the seat 2 can be modified as desired. The legs 15 carry footrests 18 mounted by rotatable joints 19 on brackets 20 which can be slid up and down the legs 15 and locked there in desired attitudes to suit a particular patient. A padded cushion 21 is provided on the seat 2.

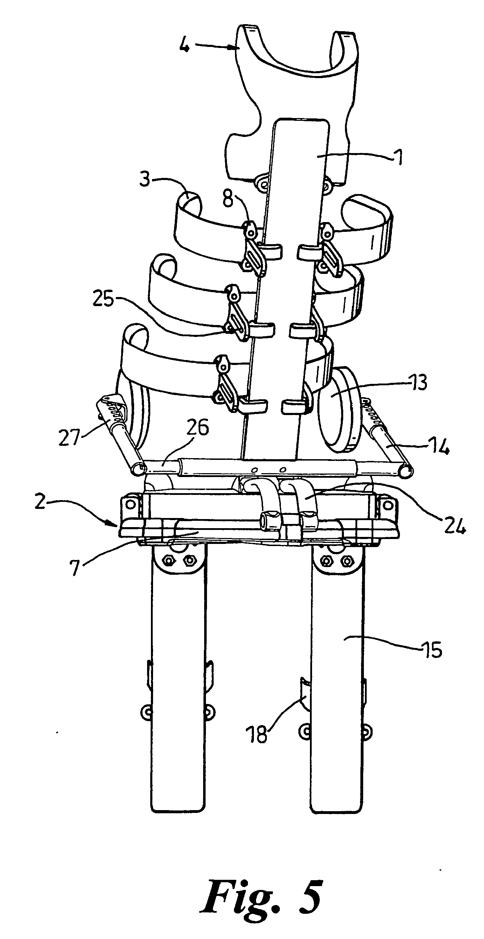

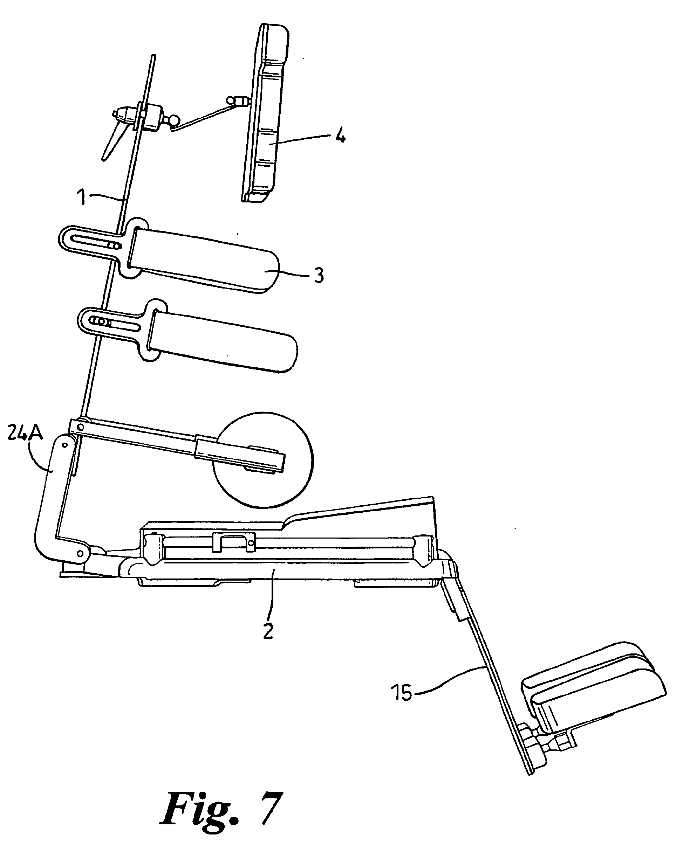

In the arrangement shown in Figures 5 and 6 parts similar to those for the examples in Figures 1 to 3 are given the same reference numerals. In this arrangement, the main difference is the mounting of the bottom end of the spine 1 onto the arcuate bar 7. This is achieved by an angled pair of elbow-shaped arms 24 which provide for a further variation in the attitude of the system. Thus, as can be seen from Figure 7, the pair of arms 24 can be moved to a new attitude 24A (or any desired intermediate position) so as both to raise the spine 1 vertically and move it rearwardly. This allows for persons of differing sizes or for the growth over time of a person using the support structure.

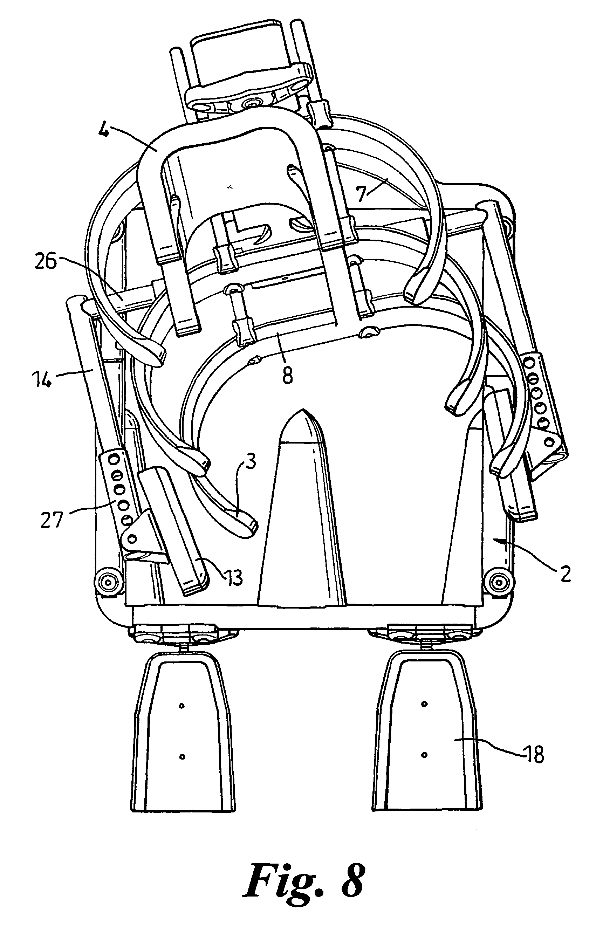

As can be seen quite clearly from Figures 5 and 6 and particularly Figure 8, the structure can be adapted in a variation of ways to suit the particular attitude of a user suffering from spinal malformation. Thus, the pairs of ribs 3 can be slid sideways relative to one another within the brackets 8 so that a sideways twist in the spine can be accommodated. Vertical displacement can be accommodated by sliding plates 25 in and out with respect to the spine 1. The headrest 4 can be rotated and raised up and down about the arm 10 to allow for the positioning of the head with respect to the rest of the body. The arms 14 carrying the pads 13 can be adjusted rotatably about a support 26 and can also be moved in and out by virtue of the telescopic portion 27 and the pads 13 themselves can be swivelled. Lastly, the whole of the back support structure can be rotated with respect to the seat 2 about the arcuate bar 7. Fixing members are provided to lock each of the parts in the final desired attitude.

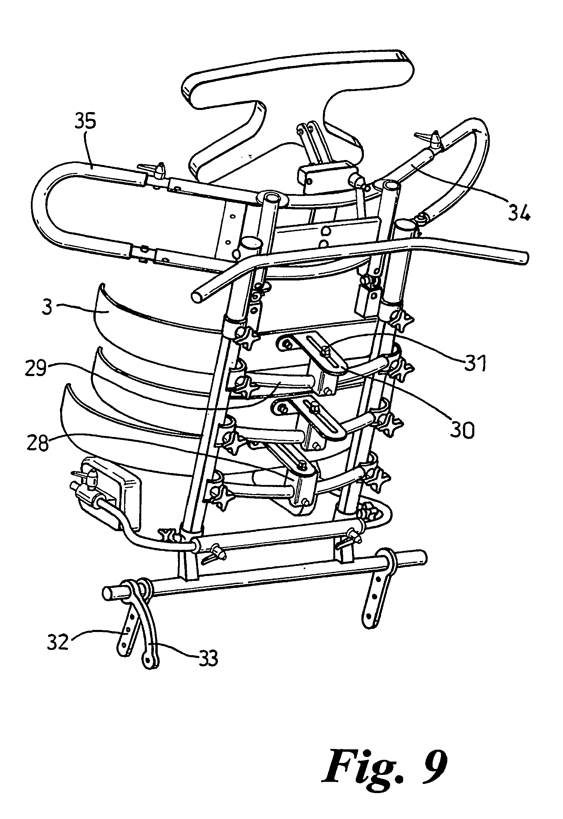

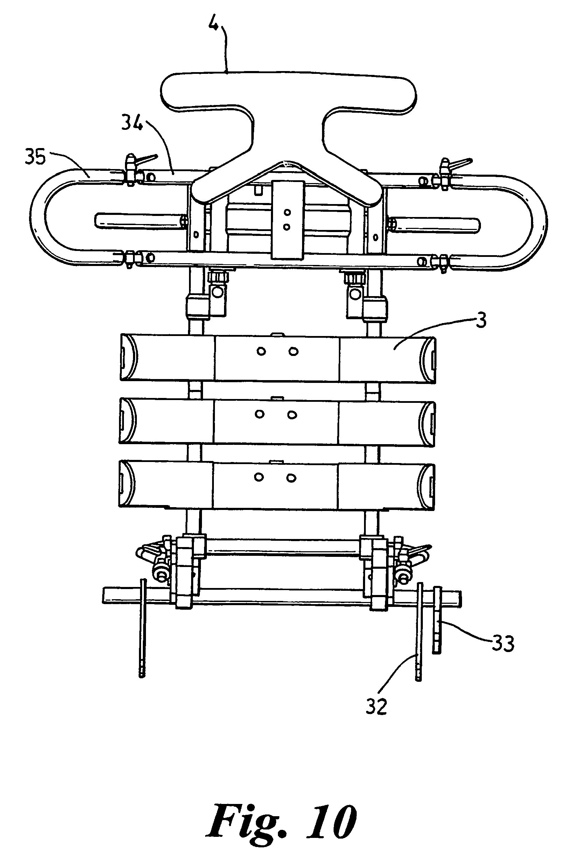

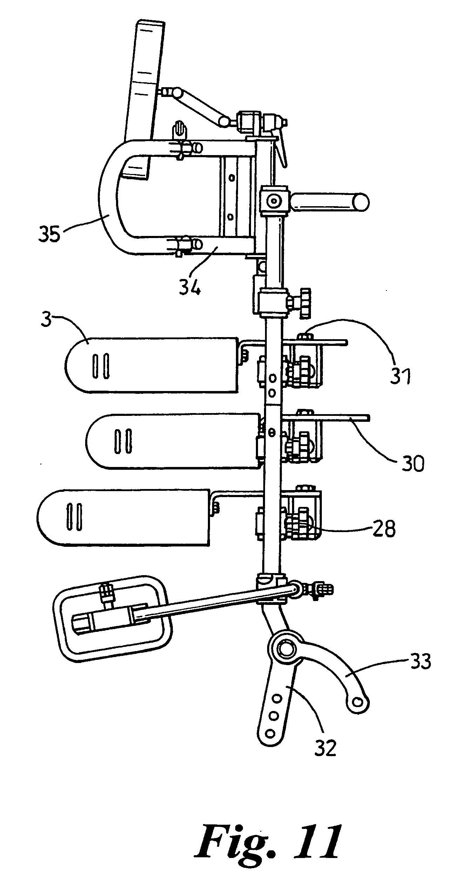

In the arrangement shown in Figures 9 to 11 the main difference is that mounting members 28 which carry the pairs of ribs 3 are individually mounted on separate curved bars 29. Again, each pair of ribs 3 can be slid in and out on a plate 30 and this plate can also be rotated with respect to a locking nut 31. This structure is designed to be mounted as a seat back on an easy chair by mounting of brackets 32, 33. The upper part of the framework carries shoulder support members 34, the end part 35 of which can be rotated and/or slid in and out to a desired position to provide a comfortable shoulder support for the user.

The structures shown in the drawings allow for multi position support of a user. There is also a degree of adjustment to allow for growth over time of the user and for modification in the attitude adopted by the body of the user. The user will be located on the seat and the pairs of ribs 3 will be adjusted to suit the adopted attitude of the user with the ends of the ribs being forced into new fixed positions as needed. Straps will be provided between the ends of the ribs of each pair to hold the user in place.

The spine of the type shown in Figures 1 and 5, for example, can be formed from a material (such as fibreglass) which can absorb energy when subjected to a sudden movement which might occur during a spasm experienced by the user. This enables the user to move to accommodate the spasm, but returns the user to the normal resting position. The leg rests 15 can also be formed from a similar material so that they too can absorb the energy of a spasm. Locating members can be provided to sit behind the leg rests 15 at a desired vertical height so as to limit the extent of the leg rest which can flex during a spasm. Similarly, a fixing bracket could be provided for the spine 1 to hold the lower portion of the spine (up to a desired height) in a fairly rigid position, thus allowing only the upper portion of the spine 1 to flex. The leg rests 15 are capable of being rotated about their mounting to allow for differing pitch angles of the legs of the user.

## Claims

1. An upper torso support structure comprising a central spine (1) and at least two left and right pairs of flexible ribs (3) curving round in front of the central spine, the ribs being capable of being set in desired attitudes to restrict movement of a patient whose torso is held by the support structure, wherein the ribs are formed from flexible material which can be bent into a desired attitude and will hold the desired attitude in order to support a patient, the support structure being **characterised in that** each pair of ribs is slidably located onto the spine to enable the pair of ribs to be moved lengthwise across the spine, thus varying the respective lengths of the two ribs as they project to the left and right sides of the spine.

2. A support structure according to claim 1, wherein the pairs of ribs are mounted so as to be able to slide adjustably towards and away from the spine or be capable of rotation with respect to the spine,

3. A support structure according to any one of claims 1 to 2. wherein the pairs of ribs are mounted so as to be able to slide adjustably up and down the spine.

4. A support structure according to any one of claims 1 to 3, wherein the upper end of the spine carries a head support (4) for the head of the patient.

5. A support structure according to claim 4, wherein the head support comprises a head rest (4) with one or two left and right pairs of flexible ribs curving round in front of the head rest, the ribs being settable in desired attitudes to restrict movement of the head of a patient with respect to the head rest.

6. A support structure according to claim 4 or claim 5, wherein the head rest is tiltable and/or rotatable with respect to the spine.

7. A support structure according to any one of claims 1 to 6, wherein each pair of ribs is in the form of a strip.

8. A support structure according to claim 7, wherein each pair of ribs is provided with a flexible strip of material (3A) lying parallel thereto, with a releasable locking arrangement being provided to lock each rib of the pair to its strip to aid in the fixing of the ribs against movement.

9. A support structure according to claim 7 or claim 8, wherein the ribs and/or the flexible strips are formed from bendable aluminium or thin steel.

10. A support structure according to any one of claims 1 to 9, wherein the material for the ribs and/or the spine is selected so as to allow for some resilience from a set position, but with "memory" such that it will tend to return to the set position after flexing,

11. A support structure according to claim 10, wherein said material comprises a robust flexible composite of plastics material or a glassfibre material.

12. A support structure according to any one of claims 1 to 6, wherein each rib comprises a row or rows of beads strung on a cable, the beads having non-straight through-passageways, together with a tensioning member to act to tension the cable within the passageways in the beads so as to lock the row of beads in a desired attitude.

13. A support structure according to any one of claims 1 to 12, wherein the ribs are provided with external padding.

14. A support structure according to any one of claims 1 to 13, wherein mountings for each pair of ribs are slidably carried by horizontally curved bars forming parts of the spine.

15. A support structure according to any one of claims 1 to 14, wherein the base of the spine is attached to a seat platform (2), preferably such that the spine can be tilted forwards and backwards with respect to the seat.

16. A support structure according to claim 15, wherein the spine is connected to the platform such that it can be rotated about the seat platform.

17. A support structure according to any one of claims 1 to 16 which is provided with mountings enabling it to be attached adjustably to an existing seat.

18. A support structure according to any one of claims 15 to 17, wherein pivoted legs (15) extend down from the front edge of the seat platform.

19. A support structure according to claim 18, wherein support members (17), to which said legs are pivoted, are slidably received within slots in the body of the seat platform.

20. A support structure according to claim 18 or claim 19, wherein height-adjustable and/or rotatable and/or laterally slidable footrests are carried by said legs.

21. A support structure according to any one of claims 1 to 20, which incorporates hip support members (13), adjustable in position, which project forwards from the sides of the lower end of the spine.

22. A support structure according to any one of claims 1 to 21, wherein shoulder support members (4) are mounted near the upper end of the spine.

23. A support structure according to claim 22, wherein the shoulder supports incorporate rotatably adjustable end sections (35).

## Patentansprüche

1. Oberkörper-Trägerstruktur, umfassend eine zentrale Wirbelsäule (1) und wenigstens zwei linke und rechte Paare flexibler Rippen (3), die vor der zentralen Wirbelsäule rund gebogen sind und in gewünschte Haltungen gebracht werden können, um die Bewegung eines Patienten, dessen Oberkörper durch die Trägerstruktur gehalten wird, zu beschränken, wobei die Rippen aus flexiblem Material geformt sind, das sich in eine gewünschte Haltung biegen läßt und das die gewünschte Haltung beibehält, um einen Patienten zu stützen, wobei sich die Trägerstruktur **dadurch kennzeichnet, daß** jedes Rippenpaar auf der Wirbelsäule verschiebbar angeordnet ist, so daß die Rippenpaare in Längsrichtung über die Wirbelsäule bewegt werden können, um dadurch die entsprechenden Längen der beiden Rippen, die zu der linken und zu der rechten Seite der Wirbelsäule vorstehen, zu variieren.

2. Trägerstruktur nach Anspruch 1, **dadurch gekennzeichnet, daß** die Rippenpaare so angebracht sind, daß sie sich durch Verschieben in Richtung auf die Wirbelsäule oder von ihr weg einstellen lassen oder in der Lage sind, sich in Bezug auf die Wirbelsäule zu drehen.

3. Trägerstruktur nach einem der Ansprüche 1 bis 3, **dadurch gekennzeichnet, daß** die Rippenpaare so angebracht sind, daß sie sich durch Aufwärts- und Abwärtsverschieben auf der Wirbelsäule einstellen lassen.

4. Trägerstruktur nach einem der Ansprüche 1 bis 3, **dadurch gekennzeichnet, daß** das obere Ende der Wirbelsäule eine Kopfstütze (4) für den Kopf des Patienten trägt.

5. Trägerstruktur nach Anspruch 4, **dadurch gekennzeichnet, daß** die Kopfstütze eine Kopfauflage (4) aufweist, wobei eines oder zwei linke und rechte Paare flexibler Rippen sich vor der Kopfauflage krümmen und die Rippen in gewünschte Haltungen einstellbar sind, um dadurch die Bewegung des Kopfes eines Patienten in Bezug auf die Körperauflage zu beschränken.

6. Trägerstruktur nach Anspruch 4 oder 5, **dadurch gekennzeichnet, daß** die Kopfauflage in Bezug auf die Wirbelsäule neigbar und / oder drehbar ist.

7. Trägerstruktur nach einem der Ansprüche 1 bis 6, **dadurch gekennzeichnet, daß** jedes der Rippenpaare die Form eines Streifens hat.

8. Trägerstruktur nach Anspruch 7, **dadurch gekennzeichnet, daß** jedes Rippenpaar mit einem flexiblen Materialstreifen (3A) versehen ist, der parallel zu ihm liegt, und daß eine lösbare Verriegelungsanordnung vorgesehen ist, um jede Rippe des Paares mit ihrem Streifen zu arretieren, um dadurch die Befestigung der Rippen gegen Bewegung zu unterstützen.

9. Trägerstruktur nach Anspruch 7 oder 8, **dadurch gekennzeichnet, daß** die Rippe und / oder die flexiblen Streifen aus einem biegbaren Aluminium oder dünnen Stahl gebildet sind.

10. Trägerstruktur nach einem der Ansprüche 1 bis 9, **dadurch gekennzeichnet, daß** das Material für die Rippen und / oder die Wirbelsäule so ausgewählt ist, daß eine gewisse Verformung aus einer eingestellten Lage ermöglicht wird, jedoch mit einem "Memory"-Effekt derart, daß das Material bestrebt ist, nach dem Biegen in die eingestellte Position zurückzukehren.

11. Trägerstruktur nach Anspruch 10, **dadurch gekennzeichnet, daß** das Material eine robuste, flexible Kunststoff-Verbindung oder ein Glasfasermaterial ist.

12. Trägerstruktur nach einem der Ansprüche 1 bis 6, **dadurch gekennzeichnet, daß** jede Rippe eine Reihe oder Reihen von auf einem Seil aufgezogener Wülste aufweist, die mit nicht geraden Durchgangskanälen versehen sind, und zwar zusammen mit einem Spannkörper, um das Seil innerhalb der Durchgangskanäle in den Wülsten unter Spannung zu setzen, so daß die Reihe von Wülsten in einer gewünschten Haltung verriegelt wird.

13. Trägerstruktur nach einem der Ansprüche 1 bis 12, **dadurch gekennzeichnet, daß** die Rippen mit einem äußeren Polster versehen sind.

14. Trägerstruktur nach einem der Ansprüche 1 bis 13, **dadurch gekennzeichnet, daß** für jedes Rippenpaar von waagerecht gebogenen Stäben, die Teile der Wirbelsäule bilden, in verschiebbaren Lage getragen wird.

15. Trägerstruktur nach einem der Ansprüche 1 bis 14, **dadurch gekennzeichnet, daß** die Basis der Wirbelsäule an einer Sitzplatte (2) angebracht ist, und zwar vorzugsweise so, daß die Wirbelsäule in Bezug auf den Sitz vorwärts und rückwärts geneigt werden kann.

16. Trägerstruktur nach Anspruch 15, **dadurch gekennzeichnet, daß** die Wirbelsäule mit der Sitzplatte so verbunden ist, daß sie um die Sitzplatte gedreht werden kann.

17. Trägerstruktur nach einem der Ansprüche 1 bis 16, **dadurch gekennzeichnet, daß** die Trägerstruktur mit Befestigungslagern versehen ist, die ihre verstellbare Anbringung an einem vorhandenen Sitz ermöglichen.

18. Trägerstruktur nach einem der Ansprüche 15 bis 17, **gekennzeichnet durch** angelenkte Beine (15), die sich von dem vorderen Rand der Sitzplatte nach unten erstrecken.

19. Trägerstruktur nach Anspruch 18, **dadurch gekennzeichnet, daß** Tragkörper (17),an denen die Beine angelenkt sind, in in dem Körper der Sitzplatte vorhandenen Schlitzen verschiebbar angeordnet.

20. Trägerstruktur nach Anspruch 18 oder 19, **dadurch gekennzeichnet, daß** von den Beinen einstellbare und / oder drehbare und / oder seitlich verschiebbare Fußauflagen getragen werden.

21. Trägerstruktur nach einem der Ansprüche 1 bis 20, **dadurch gekennzeichnet, daß** die Trägerstruktur Beckenstützkörper (13) aufweist, deren Lage einstellbar ist und die von den Seiten des unteren Endes der Wirbelsäule nach vorne vorstehen.

22. Trägerstruktur nach einem der Ansprüche 1 bis 21, **dadurch gekennzeichnet, daß** nahe dem oberen Ende der Wirbelsäule Schulterstützkörper (4) angebracht sind.

23. Trägerstruktur nach Anspruch 22, **dadurch gekennzeichnet, daß** die Schulterstützen durch Drehen einstellbare Endabschnitte (35) aufweisen.

## Revendications

1. Structure de support de torse supérieur comprenant une colonne vertébrale centrale (1) et au moins deux paires gauche et droite de côtes flexibles (3) ramenées en s'incurvant à l'avant de la colonne vertébrale centrale, les côtes étant aptes à être fixées dans des attitudes désirées pour restreindre le mouvement d'un patient dont le torse est maintenu par la structure de support, les côtes étant formées à partir d'un matériau flexible qui peut être cintré dans une attitude désirée et conservera l'attitude désirée afin de supporter un patient, la structure de support étant **caractérisée par le fait que** chaque paire de côtes est située de façon coulissante sur la colonne vertébrale pour permettre à la paire de côtes d'être déplacée selon la longueur à travers la colonne vertébrale, faisant ainsi varier les longueurs respectives des deux côtes alors qu'elles se projettent sur les côtés gauche et droit de la colonne vertébrale.

2. Structure de support selon la revendication 1, dans laquelle les paires de côtes sont montées de façon à être aptes à coulisser de manière ajustable vers et à l'opposé de la colonne vertébrale ou être aptes à tourner par rapport à la colonne vertébrale.

3. Structure de support selon l'une quelconque des revendications 1 et 2, dans laquelle les paires de côtes sont montées de façon à être aptes à coulisser de façon ajustable vers le haut et vers le bas de la colonne vertébrale.

4. Structure de support selon l'une quelconque des revendications 1 à 3, dans laquelle l'extrémité supérieure de la colonne vertébrale porte un support de tête (4) pour la tête du patient.

5. Structure de support selon la revendication 4, dans laquelle le support de tête comprend un appui-tête (4) ayant une ou deux paires gauche et droite de côtes flexibles ramenées en s'incurvant à l'avant de l'appui-tête, les côtes étant aptes à être fixées dans des attitudes désirées pour restreindre le mouvement de la tête d'un patient par rapport à l'appui-tête.

6. Structure de support selon la revendication 4 ou la revendication 5, dans laquelle l'appui-tête est apte à basculer et/ou à tourner par rapport à la colonne vertébrale.

7. Structure de support selon l'une quelconque des revendications 1 à 6, dans laquelle chaque paire de côtes se présente sous la forme d'une bande.

8. Structure de support selon la revendication 7, dans laquelle chaque paire de côtes comporte une bande flexible de matériau (3A) s'étendant parallèlement à celle-ci, un dispositif de verrouillage libérable étant prévu pour verrouiller chaque côte de la paire sur sa bande pour aider à la fixation des côtes à l'encontre d'un mouvement.

9. Structure de support selon la revendication 7 ou la revendication 8, dans laquelle les côtes et/ou les bandes flexibles sont formées à partir d'aluminium cintrable ou d'acier mince cintrable.

10. Structure de support selon l'une quelconque des revendications 1 à 9, dans laquelle le matériau pour les côtes et/ou la colonne vertébrale est choisi de façon à permettre une certaine élasticité à partir d'une position définie, mais avec une « mémoire » telle qu'après flexion, il tendra à revenir à la position définie.

11. Structure de support selon la revendication 10, dans laquelle ledit matériau comprend un composite flexible robuste de matière plastique ou un matériau à fibres de verre.

12. Structure de support selon l'une quelconque des revendications 1 à 6, dans laquelle chaque côte comprend une rangée ou des rangées de billes enfilées sur un câble, les billes ayant des passages traversants non rectilignes, conjointement avec un élément de mise en tension pour agir pour mettre en tension le câble à l'intérieur des passages dans les billes de façon à verrouiller la rangée de billes dans une attitude désirée.

13. Structure de support selon l'une quelconque des revendications 1 à 12, dans laquelle les côtes comporte un rembourrage externe.

14. Structure de support selon l'une quelconque des revendications 1 à 13, dans laquelle des montures pour chaque paire de côtes sont portées de façon coulissante par des barres incurvées horizontalement formant des parties de la colonne vertébrale.

15. Structure de support selon l'une quelconque des revendications 1 à 14, dans laquelle la base de la colonne vertébrale est attachée à une plateforme de siège (2), de préférence de telle sorte que la colonne vertébrale peut être inclinée vers l'avant et vers l'arrière par rapport au siège.

16. Structure de support selon la revendication 15, dans laquelle la colonne vertébrale est reliée à la plateforme de telle sorte qu'elle peut être amenée à tourner autour de la plateforme de siège.

17. Structure de support selon l'une quelconque des revendications 1 à 16, qui est dotée de montures lui permettant d'être fixée de façon ajustable à un siège existant.

18. Structure de support selon l'une quelconque des revendications 15 à 17, dans laquelle des pieds pivotants (15) s'étendent vers le bas à partir de la bordure avant de la plateforme de siège.

19. Structure de support selon la revendication 18, dans laquelle des éléments de support (17), sur lesquels lesdits pieds sont montés pivotants, sont reçus de façon coulissante à l'intérieur de fentes dans le corps de la plateforme de siège.

20. Structure de support selon la revendication 18 ou la revendication 19, dans laquelle des repose-pieds ajustables en hauteur et/ou aptes à tourner et/ou coulissants latéralement sont portés par lesdits pieds.

21. Structure de support selon l'une quelconque des revendications 1 à 20, qui incorpore des éléments (13) de support de hanches, ajustables en position, qui se projettent vers l'avant à partir des côtés de l'extrémité inférieure de la colonne vertébrale.

22. Structure de support selon l'une quelconque des revendications 1 à 21, dans laquelle des éléments (4) de support d'épaules sont montés près de l'extrémité supérieure de la colonne vertébrale.

23. Structure de support selon la revendication 22, dans laquelle les supports d'épaules incorporent des sections d'extrémité (35) ajustables en rotation.
